# EUROPÄISCHE PATENTSCHRIFT

(11) **EP 3 755 691 B1**
(45) Veröffentlichungstag und Bekanntmachung des Hinweises auf die Patenterteilung: **06.04.2022**
(21) Anmeldenummer: 19703112.3
(22) Anmeldetag: 11.02.2019
(51) Int. Cl.: C07D 295/023, C07D 295/027, C07C 213/10, C07C 217/08, C07C 213/02

(54) **VERFAHREN ZUR KONTINUIERLICHEN DESTILLATIVEN AUFTRENNUNG VON GEMISCHEN ENTHALTEND MORPHOLIN (MO), MONOAMINODIGLYKOL (ADG), AMMONIAK, WASSER UND METHOXYETHANOL (MOE)**
METHOD FOR THE CONTINUOUS DESTILLATIVE SEPARATION OF MIXTURES COMPRISING MORPHOLINE (MO), MONOAMINODIGLYCOL (ADG), AMMONIA, WATER AND METHOXYETHANOL (MOE)
PROCÉDÉ DE SÉPARATION PAR DISTILLATION CONTINUE DE MÉLANGES CONTENANT DE LA MORPHOLINE (MO), DU MONOAMINODIGLYCOL (ADG), DE L'AMMONIAC, DE L'EAU ET DU MÉTHOXYÉTHANOL (MOE)

(30) Priorität: 22.02.2018 EP 18158097; 27.02.2018 EP 18158951
(43) Veröffentlichungstag der Anmeldung: 30.12.2020
(73) Patentinhaber: BASF SE, 67056 Ludwigshafen am Rhein (DE)
(72) Erfinder: BUSSMANN, Oliver, 67056 Ludwigshafen (DE); KOCH, Eva, 67056 Ludwigshafen (DE); HEILIG, Manfred, 67056 Ludwigshafen (DE); PFEFFINGER, Joachim, 67056 Ludwigshafen (DE); PASTRE, Joerg, 67056 Ludwigshafen (DE); MELDER, Johann-Peter, 67056 Ludwigshafen (DE)
(74) Vertreter: BASF IP Association
(86) Internationale Anmeldenummer: PCT/EP2019/053273
(87) Internationale Veröffentlichungsnummer: WO 2019/162121

(56) Entgegenhaltungen:
- WO-A1-2008/037587
- WO-A1-2008/037589

## Beschreibung

Die vorliegende Erfindung betrifft ein Verfahren zur kontinuierlichen destillativen Auftrennung von Gemischen enthaltend Morpholin (MO), Monoaminodiglykol (ADG), Ammoniak, Wasser und Methoxyethanol [= 2-Methoxyethanol = Methylglycol] (MOE), erhalten durch Umsetzung von Diethylenglykol (DEG) der Formel 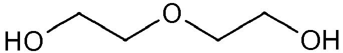 mit Ammoniak.

Aminodiglykol (ADG) [= 2-(2-Aminoethoxy)ethanol = 2,2'-Aminoethoxyethanol, Formel 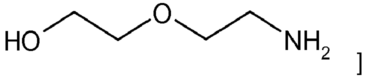 und Morpholin finden u.a. Verwendung als Lösungsmittel, Stabilisatoren, zur Synthese von Chelat-Bildnern, Kunstharzen, Arzneimitteln, Inhibitoren und grenzflächenaktiven Substanzen. N-Ethyl-morpholin (E-MO) wird u.a. als Katalysator zur Herstellung von Polyurethanschäumen verwendet.

Zur Herstellung von ADG und Morpholin sind in der Literatur zahlreiche Verfahren beschrieben.

Ullmann's Encyclopedia of Industrial Chemistry, 6th Edition, 2000 electronic release, Wiley-VCH Verlag, Rubrik 'cyclic amines' im Kapitel 'aliphatic amines' beschreibt die Synthese von ADG und MO durch Aminierung von DEG unter Wasserstoffdruck und in Gegenwart eines Kobalt- oder Nickelkatalysators (Zitate: EP-A-696 572 (BASF AG), DE-A-1 049 864) oder anderer Katalysatoren (Zitate: DE-A-3 002 342, DE-A-3 125 662 (BASF AG), US 3 155 657).

Die ältere deutsche Patentanmeldung Nr. 102005047458.6 vom 30.09.05 und die ältere europäische (Folge)Patentanmeldung Nr. 06101339.7 vom 06.02.06 (BASF AG) betreffen ein Verfahren zur Herstellung von ADG und Morpholin durch Umsetzung von DEG mit Ammoniak in Gegenwart eines spezifischen Kupfer, Nickel und Kobalt - Heterogenkatalysators sowie allgemein die Aufarbeitung durch mehrstufige Destillation.

Die beiden Patentanmeldungen WO 2008/037589 A1 und WO 2008/037590 A1 (beide BASF AG) betreffen Verfahren zur kontinuierlichen destillativen Auftrennung von Gemischen enthaltend Morpholin (MO), Monoaminodiglykol (ADG), Ammoniak und Wasser, erhalten durch Umsetzung von Diethylenglykol (DEG) mit Ammoniak.

WO 2008/037587 A1 (BASF AG) betrifft ebenfalls ein Verfahren zur kontinuierlichen destillativen Auftrennung von Gemischen enthaltend Morpholin (MO), Monoaminodiglykol (ADG), Ammoniak und Wasser, erhalten durch Umsetzung von Diethylenglykol (DEG) mit Ammoniak. Konkret wird die Destillation von rohem Morpholin bei einem Druck von 2,2 bar_{abs} offenbart.

Die WO 2008/037659 A1 betrifft ein Verfahren zur Herstellung von ADG in Elektronik-Qualität.

Die CN 102002019 A beschreibt eine destillative Methode, um Methoxyethanol aus Morpholin zu entfernen. Hierzu wird Wasserdampf in die entsprechende Destillationskolonne eingespeist. Dabei wird ausgenutzt, dass Methoxyethanol mit Wasser ein Azeotrop bildet.

Die CN 104262173 A und CN 104262177 A beschreiben beide ein Verfahren zur Umsetzung von DEG mit Ammoniak. CN 104262177 A beschreibt ferner ein Verfahren zur Aufarbeitung des zu resultierenden Gemischs.

Die Morpholin- und Monoaminodiglykolsynthese ist gekennzeichnet durch die Bildung von einer Vielzahl an Nebenkomponenten. Die Abtrennung der nicht umgesetzten Einsatzstoffe, Wert- sowie der Nebenprodukte erfolgt destillativ, was zu einem erheblichen apparativen und energetischen Aufwand führt.

Aufgrund ihrer nah beieinanderliegenden Siedepunkte ist insbesondere die Trennung von Morpholin und Methoxyethanol schwierig. Hinzukommt, dass die Anforderungen an die Reinheit von Morpholin in den letzten Jahren kontinuierlich gestiegen sind.

Der vorliegenden Erfindung lag die Aufgabe zugrunde, unter Überwindung eines Nachteils oder mehrerer Nachteile des Stands der Technik, ein verbessertes wirtschaftliches, insbesondere energieeffizientes, Verfahren zur Auftrennung von Gemischen enthaltend Morpholin (MO), Monoaminodiglykol (ADG), Ammoniak, Wasser und Methoxyethanol und ggf. N-Ethyl-morpholin (E-MO) und ggf. 1,2-Ethylendiamin (EDA) und ggf. organische Produkte mit einem Siedepunkt > 224,8°C (1,013 bar) aufzufinden. Die einzelnen organischen Komponenten (Amine), insbesondere MO und ADG und ggf. E-MO, sollten dabei in hoher Reinheit und Qualität (z.B. Farbqualität) anfallen.

Demgemäß wurde ein Verfahren zur kontinuierlichen destillativen Auftrennung von Gemischen enthaltend Morpholin (MO), Monoaminodiglykol (ADG), Ammoniak, Wasser und Methoxyethanol (MOE), erhalten durch Umsetzung von Diethylenglykol (DEG) mit Ammoniak gefunden, welches dadurch gekennzeichnet ist, dass Ammoniak, Wasser, ADG und DEG destillativ abgetrennt werden und der resultierende MO und MOE enthaltende Strom einer Destillationskolonne K40 zugeführt wird, in der bei einem Kopfdruck von 20 bis 2000 mbar über Sumpf MO, MOE und organische Produkte mit einem Siedepunkt ≥ 128 °C (1,013 bar) und über Kopf organische Produkte mit einem Siedepunkt ≤ 128 °C abgetrennt werden, sowie über einen Seitenabzug MO abgetrennt wird, wobei K40 mit einem Verdampfer zur Beheizung des Sumpfs ausgestattet ist, in den Heizdampf eingespeist wird, der einen Druck von 1 bis 10 bar aufweist.

Überraschenderweise wurde gefunden, dass in dem erfindungsgemäßen Druckbereich eine deutlich verbesserte Abtrennung von MOE möglich ist. Es ist zu vermuten, dass MOE und MO und den erfindungsgemäßen Destillationsbedingungen ein schwersiedendes Azeotrop bilden, das über Sumpf entfernt werden kann. Es kann daher beispielsweise darauf verzichtet werden, zusätzlich Wasserdampf in die K40 einzuspeisen, um MOE als Wasser/MOE Azeotrop zu entfernen.

Des Weiteren wurde gefunden, dass K40 mit Heizdampf betrieben werden kann, der einen nur geringen Druck von 1 bis 10 bar aufweist. Bevorzugt beträgt dieser Druck 1 bis 8 bar, besonders bevorzugt 1 bis 6 bar und ganz besonders bevorzugt 2 bis 5 bar oder sogar 2 bis 3 bar. Darüber hinaus wurde gefunden, dass ein solcher Heizdampf wie unten beschrieben mittels Entspannungsverdampfung eines Kondensats erhalten werden kann, das aus der Kondensation eines Heizdampfs in einem Wärmetauscher resultiert, wobei der Heizdampf vor seiner Kondensation in dem Wärmetauscher einen Druck von 2 bis 50 bar aufweist (siehe unten).

Sofern nichts anderes angegeben ist, beziehen sich die Angaben des Drucks in den jeweiligen Destillationskolonnen und Verdampfern sowie des Heizdampfs auf den Absolutdruck.

Sofern nichts anderes angegeben ist, beziehen sie die folgenden Angaben bezüglich des Drucks in den jeweiligen Destillationskolonnen auf den Kopfdruck.

Die Umsetzung von DEG mit Ammoniak erfolgt üblicherweise in einem Reaktor C1, wobei DEG und Ammoniak vor Eintritt in C1 mittels eines Wärmetauschers W2 erhitzt werden, in den Wasserdampf eingespeist wird, der einen Druck von 2 bis 50 bar, bevorzugt 3 bis 45 bar, besonders bevorzugt 4 bis 40 bar aufweist.

Die Umsetzung von DEG und Ammoniak erfolgt üblicherweise in Gegenwart von Wasserstoff und einem Heterogen-Hydrierkatalysator (im Folgenden auch als Katalysator bezeichnet). Dabei wird der Wasserstoff bevorzugt über einen Hochdruckabscheider als Kreisgas in den Reaktor zurückgeführt.

Bei der Umsetzung von DEG und Ammoniak sind insbesondere die Ausführungsformen (A) und (B) bevorzugt. Im Folgenden wird zunächst die Ausführungsform (A) und anschließend Ausführungsform B

In der bevorzugten Ausführungsform (A) beträgt der Umsatz bezogen auf DEG bevorzugt 40 bis 90 % (beispielsweise 40 bis 75%), bevorzugt 50 bis 80 % besonders bevorzugt 50 bis 75 % oder sogar 50 bis 70 % beträgt.

Bevorzugt erfolgt die Umsetzung von Diethylenglykol (DEG) mit Ammoniak in Gegenwart von Wasserstoff und einem Heterogen-Hydrierkatalysators, wobei
- der Umsatz bezogen auf DEG 40 bis 90 % (beispielsweise 40 bis 75%), bevorzugt 50 bis 80 %, besonders bevorzugt 50 bis 75 % oder sogar 50 bis 70 % beträgt,
- die Umsetzung bei einem Druck von 100 bis 300 bar und einer Temperatur von 170 °C bis 220 °C erfolgt,
- das Molverhältnis von Ammoniak zu DEG 4 bis 10 beträgt, und
- die Katalysatorbelastung im Bereich von 0,05 bis 5 kg, bevorzugt 0,1 bis 2 kg Diethylenglykol (DEG) pro Liter Katalysator (Schüttvolumen) und Stunde liegt.

Der im voranstehenden Absatz genannte Heterogen-Hydrierkatalysator enthält bevorzugt Cu, Ni und Aluminiumoxid als Träger. In einem besonders bevorzugten Heterogen-Hydrierkatalysator enthält die katalytisch aktive Masse des Katalysators vor der Behandlung mit Wasserstoff sauerstoffhaltige Verbindungen des Aluminiums, Kupfers, Nickels und Kobalts und im Bereich von 0,2 bis 5,0 Gew.-% sauerstoffhaltige Verbindungen des Zinns, berechnet als SnO. Katalysatoren dieses Typs sind beispielsweise in WO 2011/067199 A1 (BASF SE) beschrieben. Insbesondere werden Katalysatoren eingesetzt, deren katalytisch aktive Masse vor deren Reduktion mit Wasserstoff im Bereich von
15 bis 80 Gew.-% sauerstoffhaltige Verbindungen des Aluminiums, berechnet als Al₂O₃,
1 bis 20 Gew.-% sauerstoffhaltige Verbindungen des Kupfers, berechnet als CuO,
5 bis 35 Gew.-% sauerstoffhaltige Verbindungen des Nickels, berechnet als NiO,
5 bis 35 Gew.-% sauerstoffhaltige Verbindungen des Kobalts, berechnet als CoO, und
0,2 bis 5,0 Gew.-% sauerstoffhaltige Verbindungen des Zinns, berechnet als SnO, enthält.

Besonders bevorzugt erfolgt die Herstellung des im erfindungsgemäßen Verfahren eingesetzten Gemisches gemäß der WO 2011/067199 A1 (BASF SE).

Die Ausführungsform (A) bei einem Umsatz von maximal 75 % entspricht einer ADG-Iastigen Fahrweise. D.h. es wird entsprechend mehr ADG als Morpholin (MO) gebildet.

In der anderen bevorzugten Ausführungsform (B) wird Katalysator, enthaltend Cu und Ni auf Aluminiumoxid als Träger, wie insbesondere in EP-A-70 397 (BASF AG) beschrieben, eingesetzt. Katalysatoren dieses Typs sind auch in EP-A-514 692 und EP-A-167 872 (beide BASF AG) beschrieben.

In einem hierbei besonders bevorzugten Katalysator enthält die katalytisch aktive Masse des Katalysators vor der Behandlung mit Wasserstoff im Bereich von 25 bis 65 Gew.-% Aluminiumoxid (Al₂O₃), 30 bis 60 Gew.-%, sauerstoffhaltige Verbindungen des Kupfers, berechnet als CuO, und 5 bis 15 Gew.-%, sauerstoffhaltige Verbindungen des Nickels, berechnet als NiO.

Die für die Umsetzung von Diethylenglykol (DEG) mit Ammoniak bevorzugte Temperatur im Reaktor liegt hierbei im Bereich von 190-235°C. Bevorzugt ist eine isotherme Reaktorfahrweise.

Der für die Umsetzung von Diethylenglykol (DEG) mit Ammoniak bevorzugte Druck liegt im Bereich von 20 bis 30 bar.

Das Molverhältnis Ammoniak zu DEG liegt bevorzugt im Bereich von 1:1 bis 50:1.

Der DEG-Umsatz liegt bevorzugt im Bereich von 80 bis 98 %.

Die Katalysatorbelastung liegt im Allgemeinen im Bereich von 0,01 bis 2, bevorzugt 0,05 bis 0,5 kg Diethylenglykol (DEG) pro Liter Katalysator (Schüttvolumen) und Stunde.

Die Ausführungsform (B) entspricht einer MO-lastigen Fahrweise. D.h. es wird entsprechend mehr Morpholin (MO) als ADG gebildet.

Die Umsetzung gemäß Ausführungsform (A) ist gegenüber der Umsetzung gemäß Ausführungsform (B), bevorzugt.

Bevorzugt wird zur destillativen Abtrennung von Ammoniak, Wasser, ADG und DEG der Ammoniak in einer ersten Destillationskolonne K10 über Kopf abgetrennt, der Sumpfaustrag von K10 einer zweiten Destillationskolonne K20 zugeführt, in der Wasser und organische Produkte über Kopf bei einer Kopftemperatur von 45 bis 198 °C und einem Kopfdruck von 0,1 bis 15 bar abgetrennt werden, und der Sumpfaustrag von K20 einer dritten Destillationskolonne K30 zugeführt, in der MO, MOE und organische Produkte mit einem Siedepunkt < 140 °C (1,013 bar) (MO und MOE enthaltender Strom) über Kopf oder einen Seitenabzug abgetrennt werden und ADG, DEG und organische Produkte mit einem Siedepunkt > 190 °C (1,013 bar) über Sumpf abgetrennt werden.

Die in Kolonne K40 über Sumpf abgetrennten organischen Produkte werden aufgrund des hohen Gehalts an Methoxyethanol (MOE) üblicherweise entsorgt, beispielsweise verbrannt.

Die Kolonne K10 weist bevorzugt im Bereich von 3 bis 30, besonders im Bereich von 5 bis 20, theoretische Trennstufen auf.

Sie wird bevorzugt bei einem Druck im Bereich von 5 bis 30 bar, besonders 10 bis 20 bar, betrieben.

Bevorzugt befindet sich die Zulaufstelle bei Kolonne K10 im oberen Drittel, bezogen auf die Zahl der theoretischen Trennstufen.

Die Kolonne K20 weist bevorzugt im Bereich von 25 bis 70, besonders im Bereich von 30 bis 60, theoretische Trennstufen auf.

Sie wird bevorzugt bei einem Druck im Bereich von 0,1 bis 10 bar, besonders 0,8 bis 7 bar, betrieben.

Bevorzugt befindet sich die Zulaufstelle bei Kolonne K20 im mittleren Drittel, bezogen auf die Zahl der theoretischen Trennstufen.

In der Kolonne K20 wird bevorzugt Wasser abgetrennt. Mit diesem Wasser werden bevorzugt organische Produkte als Minimumazeotrop über Kopf abgetrennt, welche zum Teil höhere Siedepunkte als das Sumpfprodukt Morpholin haben.

Bei einer ADG-Iastigen Fahrweise (siehe oben) besteht das Problem, dass in den Sümpfen der Kolonnen K10 und K20 viele Hochsieder (insbesondere ADG und DEG) vorhanden sind, wodurch entsprechend hohe Sumpftemperaturen resultieren. Dies stellt insbesondere ein Problem für bereits bestehende Anlagen dar, die ursprünglich im Hinblick auf ein MO-lastige Fahrweise konzipiert wurden. Hierunter ist eine Fahrweise zu verstehen, bei der entsprechend viel Morpholin im Vergleich zu ADG hergestellt wird. Hier ist die Menge der hochsiedenden Komponenten DEG und ADG folglich deutlich geringer als bei einer ADG-Iastigen Fahrweise.

Sollen solche Anlagen nun ADG-lastig betrieben werden, so kann aufgrund des höheren Gehalts der hochsiedenden Komponenten DEG und ADG das Problem auftreten, dass der konventionell (für solche Anlagen) zur Verfügung stehende Heizdampf, welcher üblicherweise einen Druck von bis zu 50 bar aufweist, nicht mehr ausreichend ist, den Sumpf der K10 und K20 ausreichend mit Wärme zu versorgen. Des Weiteren besteht das Problem einer verminderten Produktqualität, hervorgerufen durch die hohen Sumpftemperaturen. Hinzu kommt der Nachteil, dass Heizdampf mit einem entsprechend hohen Druck (beispielsweise über 50 bar) nur sehr aufwendig, insbesondere im Hinblick auf die hierfür notwendige Energie, herstellbar ist.

Bevorzugt wird daher der Wasser und organische Produkte enthaltende Strom, der bei der Kolonne K20 über Kopf abgetrennt wird, teilweise in den Zulauf oder Sumpf der Kolonne K10 zurückgeführt. Bevorzugt werden 10 bis 80 Gew.-%, besonders bevorzugt 20 bis 70 Gew.-% zurückgeführt.

Ebenfalls bevorzugt wird der MO und MOE enthaltene Strom, der bei Kolonne K30 über Kopf abgetrennt wird und/oder dar am Seitenabzug von K40 erhaltene Strom (Morpholin), teilweise in den Zulauf der Kolonne K20 zurückgeführt. Bevorzugt werden insgesamt 20 bis 90 Gew.-%, besonders bevorzugt 25 bis 80 Gew.-% der aus K30 und/oder K40 zurückgeführten Ströme zurückgeführt. Bei einer Rückführung aus beiden Kolonnen beziehen sich die Prozentangaben des voranstehenden Satzes auf die Summe der aus K30 am Kopf und K40 am Seitenabzug erhaltenen Ströme. Eine solche Rückführung aus K30 und/oder K40 ist insbesondere dann vorteilhaft, wenn der Druck in Kolonne K20 im Bereich 1,5 bis 10 bar beziehungsweise 2 bis 7 bar liegt.

Durch die jeweilige Rückführung lässt sich die Sumpftemperatur in K10 und K20 erniedrigen. Somit lässt sich die Produktqualität verbessern und die besagten Destillationskolonnen können mit konventionell zur Verfügung stehendem Heizdampf betrieben werden. Insbesondere hat der in den Verdampfern der K10 und K20 eingespeiste Heizdampf daher einen Druck gemäß den unten für diese Kolonnen angegebenen (bevorzugten) Bereichen.

Die Kolonne K30 weist bevorzugt im Bereich von 5 bis 25, besonders im Bereich von 7 bis 20, theoretische Trennstufen auf.

Sie wird bevorzugt bei einem Druck im Bereich von 0,01 bis 5 bar, besonders 0,1 bis 2,5 bar, betrieben.

Bevorzugt befindet sich die Zulaufstelle bei Kolonne K30 im oberen Drittel, bezogen auf die Zahl der theoretischen Trennstufen.

In der alternativen Ausführungsform befindet sich der Seitenabzug bevorzugt 1 bis 8 theoretische Trennstufen, besonders 2 bis 6 theoretische Trennstufen, oberhalb der Zulaufstelle.

Die Kolonne K40 weist bevorzugt im Bereich von 10 bis 80, besonders im Bereich von 15 bis 60, theoretische Trennstufen auf.

Sie wird bevorzugt bei einem Druck im Bereich von 30 bis 1500 mbar, besonders bevorzugt 50 bis 800 mbar und ganz besonders bevorzugt 80 bis 750 mbar beispielsweise 100 bis 500 mbar oder sogar 100 bis 450 mbar, betrieben.

Bevorzugt befindet sich die Zulaufstelle bei Kolonne K40 im oberen oder mittleren, besonders mittleren, Drittel, bezogen auf die Zahl der theoretischen Trennstufen.

Der gegenüberliegende MO Seitenabzug befindet sich bevorzugt 1 bis 30 theoretische Trennstufen, besonders 2 bis 25 theoretische Trennstufen, unterhalb der Zulaufstelle.

In der Kolonne K40 werden organische Produkte mit einem Siedepunkt ≤ 128°C (1,013 bar), bevorzugt < 128°C (1,013 bar), wie z.B. EDA, über Kopf abgetrennt und organische Produkte mit einem Siedepunkt ≥ 128 °C (1,013 bar) über Sumpf abgetrennt.

Die in der Kolonne K40 über Kopf abgetrennten organischen Produkte, insbesondere EDA, können vorteilhaft ganz oder teilweise in den Zulauf zur Kolonne K20 zurückgeführt werden.

In einer weiteren Ausführungsform kann durch weitere destillative Aufreinigung des Kopfdestillats reines EDA als Wertprodukt gewonnen werden.

In einer besonderen Ausführungsform wird der wasser- und organische Produkte enthaltende Strom, der bei der Kolonne K20 über Kopf abgetrennt wird, einer Kolonne K50 zugeführt, in der wässrige N-Ethyl-morpholin-Lösung (wässrige E-MO-Lösung) über Kopf oder einen flüssigen Seitenabzug, wobei sich der flüssige Seitenabzug bevorzugt im oberen Drittel der Kolonne, bezogen auf die Zahl der theoretischen Trennstufen, befindet, und Wasser über Sumpf abgetrennt wird.

Die Kolonne K50 weist bevorzugt im Bereich von 10 bis 50, besonders im Bereich von 15 bis 40, theoretischen Trennstufen auf.

Sie wird bevorzugt bei einem Druck im Bereich von 0,1 bis 16 bar, besonders 0,2 bis 8 bar, betrieben.

Bevorzugt befindet sich die Zulaufstelle bei Kolonne K50 im oberen oder mittleren, besonders im mittleren, Drittel, bezogen auf die Zahl der theoretischen Trennstufen.

Die wässrige N-Ethyl-morpholin-Lösung wird zur Gewinnung des reinen E-MOs zunächst entwässert. Als entwässerndes Mittel wird bevorzugt Natronlauge, z.B. als 40-60 Gew.-%ige wässrige Lösung, besonders 50 Gew.-%ige wässrige Lösung, eingesetzt. Bevorzugt wird die Entwässerung mit der Natronlauge kontinuierlich in einer Extraktionskolonne durchgeführt. Die Extraktionstemperatur liegt bevorzugt zwischen 25-60°C, besonders zwischen 30-55°C. Die Natronlauge wird dabei auf 15-35 Gew.-%, besonders 20-30 Gew.-%, verdünnt.

Nach der Phasentrennung wird die organische Phase in einer kontinuierlichen oder diskontinuierlichen Destillation aufgearbeitet. Die Destillation wird bevorzugt diskontinuierlich in einer Destillationsblase durchgeführt.

Die Kopfprodukte fallen dabei nacheinander an: ggf. Ethylamin, ggf. Ethanol als wässriges Azeotrop, ggf. N-Methyl-morpholin als wässriges Azeotrop, ggf. wasserfreies N-Methyl-morpholin und das Wertprodukt N-Ethyl-morpholin (E-MO).

In einer bevorzugten Ausführungsform wird der Sumpfaustrag von K30 einer Destillationskolonne K60 zugeführt, in der ADG in einem Seitenabzug abgetrennt wird, organische Produkte mit einem Siedepunkt ≤ 224,8°C (1,013 bar) über Kopf abgetrennt und organische Produkte mit einem Siedepunkt > 255°C (1,013 bar) über Sumpf abgetrennt werden.

Die Kolonne K60 weist bevorzugt im Bereich von 20 bis 80, besonders im Bereich von 30 bis 70, theoretische Trennstufen auf.

Sie wird bevorzugt bei einem Druck im Bereich von 0,005 bis 1 bar, besonders 0,01 bis 0,7 bar, betrieben.

Bevorzugt befindet sich die Zulaufstelle bei Kolonne K60 im mittleren oder unteren, besonders mittleren, Drittel, bezogen auf die Zahl der theoretischen Trennstufen.

Bevorzugt befindet sich der gegenüberliegende ADG Seitenabzug 1 bis 30, besonders 2 bis 20, theoretische Trennstufen oberhalb der Zulaufstelle.

In einer bevorzugten Ausführungsform werden in der Kolonne K60 über Kopf abgetrennte organische Produkte, wie z.B. N-(2-Aminoethyl)morpholin, 2-(2-Aminoethoxy)ethylamin, in die Umsetzung von DEG mit Ammoniak zurückgeführt.

Um Aufpegelungen einzelner Komponenten im Kreislauf der Produktionsanlage zu vermeiden, wird bevorzugt ein Teilstrom des im Kolonnenkopf abgetrennten Destillates ausgeschleust. Der Anteil des zurückgeführten Stromes beträgt bevorzugt 40-100 Gew.-%, besonders bevorzugt 50-100 Gew.-%, des im Kolonnenkopf abgetrennten Destillates.

Der ADG enthaltende Strom, der bei der Kolonne K60 im Seitenabzug abgetrennt wird, wird bevorzugt einer Kolonne K70 zugeführt, in der ADG über einen Seitenabzug abgetrennt wird, organische Produkte mit einem Siedepunkt ≥ 224,8°C (1,013 bar), besonders > 235°C (1,013 bar), über Sumpf und organische Produkte mit einem Siedepunkt ≤ 224,8°C (1,013 bar) über Kopf abgetrennt werden.

Die Kolonne K70 weist bevorzugt im Bereich von 10 bis 80, besonders im Bereich von 20 bis 70, theoretische Trennstufen auf.

Sie wird bevorzugt bei einem Druck im Bereich von 0,005 bis 1 bar, besonders 0,01 bis 0,7 bar, betrieben.

Bevorzugt befindet sich die Zulaufstelle bei Kolonne K70 im oberen oder mittleren, bevorzugt mittleren, Drittel, bezogen auf die Zahl der theoretischen Trennstufen.

Bevorzugt befindet sich der gegenüberliegende ADG Seitenabzug 1 bis 30, besonders 2 bis 25, theoretische Trennstufen oberhalb der Zulaufstelle.

Bevorzugt werden in Kolonne K70 über Sumpf abgetrennte Produkte, wie z.B. DEG, Morpholylaminodiglykol, Morpholinodiglykol, in die Umsetzung von DEG mit Ammoniak zurückgeführt. (Morpholylaminodiglykol = 4-(2-(2-Aminoethoxy)-ethyl)-morpholin, C₈H₁₈N₂O₂; Morpholinodiglykol (Morpholinylethoxyethanol) CAS-Nr. 3603-45-0, C₈H₁₇NO₃)

Bevorzugt werden in Kolonne K70 über Kopf abgetrennte Produkte, wie z.B. ADG, N-(2-Aminoethyl)morpholin, 2-(2-Aminoethoxy)ethylamin, in die Umsetzung von DEG mit Ammoniak zurückgeführt.

Der Anteil des zurückgeführten Stromes beträgt bevorzugt 80-100 Gew.-%, besonders bevorzugt 95-100 Gew.-%, des im Kolonnenkopf abgetrennten Destillates.

In einer weiteren besonders bevorzugten Ausführungsform handelt es sich bei der Kolonne K60 um eine Trennwandkolonne (TK).

Die Trennwandkolonne (TK) weist bevorzugt eine Trennwand (T) in Kolonnenlängsrichtung unter Ausbildung eines oberen gemeinsamen Kolonnenbereichs (1), eines unteren gemeinsamen Kolonnenbereichs (6), eines Zulaufteils (2, 4) mit Verstärkungsteil (2) und Abtriebsteil (4), sowie eines Entnahmeteils (3, 5) mit Verstärkungsteil (3) und Abtriebsteil (5) auf, wobei die Zuführung des Sumpfaustrags von K30 im oberen oder mittleren Drittel, besonders oberen Drittel, des Zulaufteils (2, 4), bezogen auf die Zahl der theoretischen Trennstufen des Zulaufteils, die Abführung von organischen Produkten mit einem Siedepunkt > 255°C (1,013 bar) über Sumpf, die Abführung von organischen Produkten mit einem Siedepunkt ≤ 224,8°C (1,013 bar) über Kopf, die Abführung von ADG aus dem Kolonnenbereich 1 und optional, in einer besonderen Ausführungsform bevorzugt, die Abführung von dampfförmigen organischen Produkten mit einem Siedepunkt ≥ 224,8°C (1,013 bar), besonders > 235°C (1,013 bar), wie z.B. DEG, aus dem oberen oder mittleren Drittel, besonders oberen Drittel, des Entnahmeteils (3, 5) (Seitenabzug), bezogen auf die Zahl der theoretischen Trennstufen des Entnahmeteils, erfolgt.

In der Kolonne K60 über den dampfförmigen Seitenabzug abgetrennte organische Produkte, wie z.B. DEG, werden bevorzugt in die Umsetzung von DEG mit Ammoniak zurückgeführt.

In einer weiteren vorteilhaften Ausführungsform weist die Trennwandkolonne (TK) eine Trennwand (T) in Kolonnenlängsrichtung unter Ausbildung eines oberen gemeinsamen Kolonnenbereichs (1) und (2), eines Zulaufteils (3, 4) mit Verstärkungsteil (3) und Abtriebsteil (4), sowie eines Teils (5) auf, wobei die Trennwand T bis zum Boden der Kolonne ausgebildet ist, wobei die Zuführung des Sumpfaustrags von K30 im oberen oder mittleren Drittel, besonders oberen Drittel, des Zulaufteils (3, 4), bezogen auf die Zahl der theoretischen Trennstufen des Zulaufteils, die Abführung von DEG und organischen Produkten mit einem Siedepunkt ≥ 224,8°C (1,013 bar), bevorzugt > 235°C (1,013 bar), über Sumpf unterhalb des Teils 5, die Abführung von organischen Produkten mit einem Siedepunkt > 255°C (1,013 bar) (Hochsieder = HS) über Sumpf unterhalb der Teile 3 und 4, die Abführung von organischen Produkten mit einem Siedepunkt ≤ 224,8°C (1,013 bar) über Kopf und die Abführung von ADG aus dem mittleren Teil des oberen gemeinsamen Kolonnenbereichs (1) und (2) (Seitenabzug) erfolgt.

Die Trennwandkolonne K60 weist bevorzugt im Bereich von 30 bis 100, besonders im Bereich von 40 bis 90, theoretische Trennstufen auf.

Sie wird bevorzugt bei einem Druck im Bereich von 0,005 bis 1 bar, besonders 0,01 bis 0,7 bar, betrieben.

Der durch die Trennwand (T) unterteilte Teilbereich der Kolonne TK bestehend aus den Teilbereichen 3, 4 und 5, bzw. 2, 3, 4, und 5, oder jeweils Teilen davon ist bevorzugt mit geordneten Packungen, Füllkörpern und/oder Böden bestückt. Die Trennwand in diesen Teilbereichen ist bevorzugt wärmeisolierend ausgeführt.

In einer bevorzugten Ausführungsform werden in der Kolonne K60 über Kopf abgetrennte organische Produkte, wie z.B. N-(2-Aminoethyl)morpholin, 2-(2-Aminoethoxy)ethylamin, nicht ausgeschleust, sondern in die Umsetzung von DEG mit Ammoniak zurückgeführt.

In Kolonne K60, mit bis zum Boden der Kolonne ausgebilder Tennwand T, über Sumpf abgetrennte Produkte unterhalb des Teils 5 abgetrennte organische Produkte, wie z.B. DEG, werden bevorzugt in die Umsetzung von DEG mit Ammoniak zurückgeführt.

Der Anteil des zurückgeführten Stromes beträgt bevorzugt 80-100 Gew.-%, besonders bevorzugt 95-100 Gew.-%, des im Kolonnenkopf abgetrennten Destillates.

Das erfindungsgemäße Verfahren ist in besonderen Ausführungsformen bei Einsatz einer Trennwandkolonne (TK) vorteilhaft durch einen geringen Wärmebedarf gegenüber der 2- bzw. 3-Kolonnenverschaltung (K60 - K70 bzw. K80) sowie durch die Reduzierung der Kolonnenanzahl.

In einer besonderen Ausgestaltung des erfindungsgemäßen Verfahrens wird/werden der oder die ADG enthaltende/n Strom/Ströme, der/die bei den Kolonnen K60 und/oder K70 über Kopf abgetrennt wird/werden, ganz oder teilweise einer Kolonne K80 zugeführt, in der ADG und organische Produkte mit einem Siedepunkt ≥ 224,8°C (1,013 bar) über Sumpf und organische Produkte mit einem Siedepunkt ≤ 224,8°C (1,013 bar) über Kopf abgetrennt werden.

Das im Sumpf anfallende ADG kann als Wertprodukt verwertet werden.

Bevorzugt wird in der Kolonne K80 ADG in besonders reiner Form zusätzlich über einen Seitenabzug abgetrennt.

In diesem Fall werden in Kolonne K80 über Sumpf abgetrennte Produkte bevorzugt in den Zulauf der Kolonnen K60 und/oder K70 zurückgeführt.

In Kolonne K80 über Kopf abgetrennte Produkte werden bevorzugt in die Umsetzung von DEG mit Ammoniak zurückgeführt.

Um Aufpegelungen einzelner Komponenten im Kreislauf der Produktionsanlage zu vermeiden, wird bevorzugt ein Teilstrom des im Kolonnenkopf abgetrennten Destillates ausgeschleust. Der Anteil des zurückgeführten Stromes beträgt bevorzugt 0-100 Gew. %, besonders bevorzugt 0-50 Gew.-%, des im Kolonnenkopf abgetrennten Destillates.

Die Kolonne K80 weist bevorzugt im Bereich von 10 bis 80, besonders im Bereich von 15 bis 60, theoretische Trennstufen auf.

Sie wird bevorzugt bei einem Druck im Bereich von 0,005 bis 3 bar, besonders 0,01 bis 2 bar, betrieben.

Bevorzugt befindet sich die Zulaufstelle bei Kolonne K80 im oberen oder mittleren, bevorzugt oberen, Drittel, bezogen auf die Zahl der theoretischen Trennstufen.

Bevorzugt befindet sich der gegenüberliegende ADG Seitenabzug 1 bis 30, besonders 2 bis 25, theoretische Trennstufen unterhalb der Zulaufstelle.

In einer bevorzugten Ausführungsform wird der organische Produkte enthaltene Strom, der bei Kolonne K60 über Sumpf abgetrennt wird, einem Verdampfer V2 zugeführt, in dem Morpholinaminodiglykol, Morpholindiglykol und DEG gasförmig abgetrennt werden. Diese können beispielsweise für die Herstellung von Dimorpholindiethylether (DMDEE) eingesetzt. Der Verdampfer V1 wird bevorzugt bei einem Druck von 2 bis 25 mbar betrieben. Als Verdampfer V2 können jeweils Naturumlaufverdampfer, Zwangsumlaufverdampfer, Zwangsumlaufentspannungsverdampfer, Fallfilmverdampfer, Robertverdampfer, Kettle-Type-Verdampfer, Dünnschichtverdampfer oder Kletterfilmverdampfer eingesetzt werden. Bevorzugt werden jeweils Naturumlaufverdampfer, Zwangsumlaufverdampfer, Zwangsumlaufentspannungsverdampfer, Fallfilmverdampfer, Robertverdampfer oder Kettle-Type-Verdampfer eingesetzt. Besonders bevorzugt ist ein Dünnschichtverdampfer.

Die Kolonnen K10 bis K80 sind zur Beheizung des Sumpfs typischerweise mit einem Verdampfer ausgestattet. Als Verdampfer können jeweils Naturumlaufverdampfer, Zwangsumlaufverdampfer, Zwangsumlaufentspannungsverdampfer, Fallfilmverdampfer, Robertverdampfer, Kettle-Type-Verdampfer, Dünnschichtverdampfer oder Kletterfilmverdampfer eingesetzt werden. Bevorzugt werden jeweils Naturumlaufverdampfer, Zwangsumlaufverdampfer, Zwangsumlaufentspannungsverdampfer, Fallfilmverdampfer, Robertverdampfer oder Kettle-Type-Verdampfer eingesetzt.

Die Destillationskolonnen K10, K20, K30, K50, K60 und K70 sind zur Beheizung des Sumpfs bevorzugt mit einem Verdampfer ausgestattet, in den Heizdampf eingespeist wird, der einen Druck von 2 bis 50 bar, bevorzugt 3 bis 45 bar und besonders bevorzugt 4 bis 40 bar, aufweist.

Das erfindungsgemäße Verfahren ist in besonderen Ausführungsformen durch folgende Wärmeintegrationsmaßnahmen zusätzlich vorteilhaft:
Die Wärme aus den Brüden der K80 kann in der K50 integriert werden.

Die Wärme aus den Brüden der K70 kann in der K50 und/oder K80 integriert werden, bevorzugt in der K 50.

Die Wärme aus den Brüden der K60 kann in der K50 integriert werden.

Die Wärme aus den Brüden der K40 kann in der K20, K50 und/oder K80 integriert werden.

Die Wärme aus den Brüden der Trennwandkolonne K60 kann in der K50 integriert werden.

Diese Wärmeintegration kann wie folgt ausgeführt werden:
Um die anfallende Wärme der Brüden maximal nutzen zu können, wird bevorzugt auf ein Wärmeträgermedium verzichtet und die Brüdenströme werden bevorzugt direkt in den entsprechenden Verdampfern, anstatt des Heizdampfes, kondensiert. Bevorzugt sind die oben genannten Verdampfer. Die Restbrüden werden bevorzugt jeweils in einem Nachkondensator verflüssigt.

Weiterhin ist es vorteilhaft, die Reaktionswärme aus der Synthese des aufzutrennenden Gemisches abzuführen, insbesondere über Siedekühlung (Wasserdampf), und in der Destillation zu integrieren. Bei der Synthese sind insbesondere die Ausführungsformen (A) und (B), insbesondere Ausführungsform (A) bevorzugt.

Die Reaktionswärme kann hierbei in den Kolonnen K20, K50, K30, K40, K70 und/oder K80, bevorzugt in den Kolonnen K20, K40 und/oder K80 integriert werden.

In einer weiteren bevorzugten Ausführungsform betreffend die Wärmeintegration wird Heizdampf für die Kolonne K40 mittels Entspannungsverdampfung eines Kondensats erhalten, das aus der Kondensation eines Heizdampfs in einem Wärmetauscher resultiert, wobei der Heizdampf vor seiner Kondensation in dem Wärmetauscher einen Druck von 2 bis 50 bar, bevorzugt 3 bis 45 bar, besonders bevorzugt 4 bis 40 bar aufweist. Zwangsläufig ist dabei der Druck des Heizdampfs, der in den Wärmetauscher eingespeist wird, größer als der Druck des mittels Entspannungsverdampfung gewonnen Heizdampfs, der in den Verdampfer der K40 eingespeist wird. Diese Art der Wärmeintegration wird dadurch ermöglicht, dass die Kolonne bei dem geringen, erfindungsgemäßen Druck betrieben wird. Erfindungsgemäß kann der Heizdampf für die Kolonne K40 vollständig oder teilweise mittels Entspannungsverdampfung eines entsprechenden Kondensats erhalten werden. Es ist möglich, dass ein Teil der benötigten Restwärme durch Wasserdampf aus dem Netz (Druckbereich: 1 bis 10 bar) bereitgestellt wird. Üblicherweise wird mindestens 50%, bevorzugt mindestens 60 %, besonders bevorzugt mindestens 70 % und ganz besonders bevorzugt mindestens 80 % oder sogar mindestens 90 % des benötigten Heizdampfs mittels Entspannungsverdampfung gewonnen. Ebenso ist es möglich, dass der Heizdampf vollständig mittels Entspannungsverdampfung gewonnen wird.

Technisch wird die Entspannungsverdampfung üblicherweise so realisiert, dass der im Wärmetauscher kondensierte Dampf in einen geeigneten Kessel (teilweise) entspannt wird. Hierdurch verdampft das Kondensat und steht erneut als Heizdampf zur Verfügung. Der so gewonnene Heizdampf hat zwangsläufig einen geringeren Druck als der ursprünglich in den Wärmetauscher eingespeiste.

Als Wärmetauscher kommt grundsätzlich jeder beliebige Wärmetauscher in Frage, der mit Heizdampf betrieben wird, der einen entsprechenden Druck aufweist. Es muss sich daher nicht um einen Wärmetauscher handeln, der zu dem erfindungsgemäßen Verfahren gehört. Bevorzugt handelt es sich um den Wärmetauscher W2 (siehe unten) oder den Verdampfer einer der Kolonnen K10, K20, K30, K50, K60 oder K70. Die Erfindung ist zudem nicht derart beschränkt, dass die Wärmeintegration mit genau einem Wärmetauscher (bspw. K10) erfolgt. In Abhängigkeit von der in K40 benötigten Wärmemenge ist es auch möglich, dass die Wärmeintegration in K40 aus zwei oder mehreren Wärmetauschern (bspw. K10 und K20) erfolgt. Ebenso ist es möglich, dass noch benötigte Restwärme wie oben beschrieben durch Wasserdampf aus dem Netz bereitgestellt wird.

In einer bevorzugten Ausführungsform wird das Reaktionsgemisch der Umsetzung von DEG mit Ammoniak vor Zuführung in die Kolonne K10 einem Verdampfer V1 zugeführt, in dem ein Teil des Ammoniaks, bevorzugt 20 bis 80 Gew.-% des im Zuführstrom enthaltenden Ammoniaks, gasförmig abgetrennt wird. Der Verdampfer V1 wird bevorzugt bei einem Druck von 14 bis 25 bar betrieben. Es können dieselben Verdampfer wie die oben für V2 genannten verwendet werden. Ganz besonders bevorzugt handelt es sich bei V2 um einen Kettle-Type-Verdampfer. Bevorzugt wird in den Verdampfer V1 Heizdampf eingespeist, der einen Druck von 1 bis 10 bar, bevorzugt 1 bis 8 bar, besonders bevorzugt 1 bis 6 bar oder sogar 2 bis 5 bzw. 2 bis 3 aufweist.

In einer besonders bevorzugten Ausführungsform wird Heizdampf für den Verdampfer V1 mittels Entspannungsverdampfung eines Kondensats erhalten, das aus der Kondensation eines Heizdampfs in einem Wärmetauscher resultiert, wobei der Heizdampf vor seiner Kondensation in dem Wärmetauscher einen Druck von 2 bis 50 bar, 3 bis 45 bar, besonders bevorzugt 4 bis 40 bar, aufweist. Zwangsläufig ist dabei der Druck des Heizdampfs, der in den Wärmetauscher eingespeist wird, größer als der Druck des mittels Entspannungsverdampfung gewonnen Heizdampfs, der in den Verdampfer V1 eingespeist wird. Erfindungsgemäß kann der Heizdampf für den Verdampfer V1 vollständig oder teilweise mittels Entspannungsverdampfung eines entsprechenden Kondensats erhalten werden. Es ist möglich, dass ein Teil der benötigten Restwärme durch Wasserdampf aus dem Netz (Druckbereich: 1 bis 10 bar) bereitgestellt wird. Üblicherweise wird mindestens 50%, bevorzugt mindestens 60 %, besonders bevorzugt mindestens 70 % und ganz besonders bevorzugt mindestens 80 % oder sogar mindestens 90 % des benötigten Heizdampfs mittels Entspannungsverdampfung gewonnen. Ebenso ist es möglich, dass der Heizdampf vollständig mittels Entspannungsverdampfung gewonnen wird.

Als Wärmetauscher kommt grundsätzlich jeder beliebige Wärmetauscher in Frage, der mit Heizdampf betrieben wird, der einen entsprechenden Druck aufweist. Es muss sich daher nicht um einen Wärmetauscher handeln, der zu dem erfindungsgemäßen Verfahren gehört. Bevorzugt handelt es sich bei dem Wärmetauscher um den Wärmetauscher W2 oder den Verdampfer einer der Kolonnen K10, K20, K30, K50, K60 oder K70. Die Erfindung ist zudem nicht derart beschränkt, dass die Wärmeintegration mit genau einem Wärmetauscher (bspw. K10) erfolgt. In Abhängigkeit von der in V1 benötigten Wärmemenge ist es auch möglich, dass die Wärmeintegration in V1 aus zwei oder mehreren Wärmetauschern (bspw. K10 und K20) erfolgt. Ebenso ist es möglich, dass noch benötigte Restwärme wie oben beschrieben durch Wasserdampf aus dem Netz bereitgestellt wird.

Im Übrigen gilt das oben bezüglich der Wärmeintegration in K40 gesagte hier entsprechend.

In solchen Ausführungsformen, in denen sowohl eine Wärmeintegration in K40 als auch in den Verdampfer V1 erfolgt, kann der mittels Entspannungsverdampfung gewonnene Heizdampf sowohl aus demselben als auch aus unterschiedlichen Wärmetauschern stammen. Beispielsweise kann eine Wärmeintegration in K40 und V1 ausschließlich aus dem Verdampfer der K10 erfolgen. Ebenso ist es möglich, dass die Wärmeintegration in K40 aus dem Verdampfer der K10 und die Wärmeintegration in V1 aus dem Verdampfer der K20 erfolgt.

Das erfindungsgemäße Verfahren ist insbesondere vorteilhaft zur Herstellung von Morpholin (MO)
mit einer Reinheit von ≥ 99,5 Gew.-%, besonders ≥ 99,6 Gew.-%, z.B. 99,65 bis 99,95 Gew.-%, einem Gehalt an N-Ethyl-morpholin (E-MO) von ≤ 0,20 Gew.-%, besonders ≤ 0,10 Gew.-%, z.B. 0,01 bis 0,08 Gew.-%,
einem Gehalt an 1,2-Ethylendiamin (EDA) von ≤ 0,30 Gew.-%, besonders ≤ 0,20 Gew.-%, z.B. 0,05 bis 0,15 Gew.-%,
einem Gehalt an 2-Methoxy-ethanol (MOE) von < 0,3 Gew.-%, besonders ≤ 0,10 Gew.-%, insbesondere 0,04 Gew.-% bis 0,1 Gew.-%,
und einem Gehalt an Wasser von ≤ 0,05 Gew.-%, besonders ≤ 0,04 Gew.-%, z.B. 0,01 bis 0,03 Gew.-%.

Ganz besonders ist es vorteilhaft zur Herstellung von Morpholin (MO) mit einer APHA-Farbzahl von ≤ 10, besonders ≤ 8, z.B. 2 bis 7,
und einem Chloridgehalt von ≤ 15 mg/Liter, besonders ≤ 5 mg/Liter, ganz besonders ≤ 1 mg/Liter, z.B. 0,1 bis 0,9 mg/Liter.

Das erfindungsgemäße Verfahren ist weiter insbesondere vorteilhaft zur Herstellung von Monoaminodiglykol (ADG) mit einer Reinheit von ≥ 98,00 Gew.-%, besonders ≥ 98,30 Gew.-%, z.B. 98,50 bis 99,50 Gew.-%,
einem Gehalt an DEG von ≤ 0,40 Gew.-%, besonders ≤ 0,10 Gew.-%, z.B. 0,01 bis 0,08 Gew.-%,
einem Gehalt an Wasser von ≤ 0,20 Gew.-%, besonders ≤ 0,10 Gew.-%, z.B. 0,01 bis 0,08 Gew.-%,
und einer APHA-Farbzahl von ≤20, besonders ≤ 15, ganz besonders ≤ 10, z.B. 2 bis 8.

Das erfindungsgemäße Verfahren ist weiter insbesondere vorteilhaft zur Herstellung von N-Ethyl-morpholin (E-MO) mit einer Reinheit von ≥ 98,50 Gew.-%, besonders ≥ 99,00 Gew.-%, z.B. 99,50 bis 99,90 Gew.-%,
einem Gehalt an Wasser von ≤ 0,30 Gew.-%, besonders ≤ 0,20 Gew.-%, z.B. 0,05 bis 0,15 Gew.-%,
und einer APHA-Farbzahl von ≤ 50, besonders ≤20, ganz besonders ≤ 10, z.B. 2 bis 8.

APHA-Farbzahlen werden bestimmt gemäß DIN EN 1557.

Der Wassergehalt wird bestimmt gemäß DIN 51777 (K. Fischer).

Der Chloridgehalt wird mittels lonenchromatographie bestimmt (Detektion von Leitfähigkeit mit chemischer Suppression), nach folgender Methode:
Probenvorbereitung: Ca. 2 g Probe werden in einen Messkolben (10 ml) eingewogen und mit Eluent bis zur Marke aufgefüllt.

Messbedingungen:
lonenchromatographie-System: Metrohm Modulares System (733)
Vorsäule: z.B. DIONEX AG 12; Trennsäule: z.B. DIONEX AS 12
Eluent: z.B. 2,7 mmol Na₂CO₃, 0,28 mmol/I NaHCO₃ in Wasser
Fluss: 1 ml/min; Dosiervolumen: 100 µl
Detektion: Leitfähigkeit nach chemischer Suppression
Suppressor: Metrohm Modul 753
Regenerent: 50 mmol H₂SO₄ in Reinstwasser, (Fluss ca. 0,4 ml/min)
Kalibration: Extern, überprüft durch Aufstock-Versuche
Bestimmungsgrenze: 0,1 mg/kg Chlorid in der Probe.

Im Wertprodukt Morpholin wird der Gehalt an Morpholin, 1,2-Ethylendiamin, N-Ethyl-morpholin, 2-Methoxy-ethanol mittels GC bestimmt (GC-Bedingungen: 30 m DB-1; Temperaturprogramm mit 60°C Anfangstemperatur, 4°C/min. Heizrate, 190°C Endtemperatur).

Im Wertprodukt ADG wird der Gehalt an ADG und DEG in mittels GC bestimmt (GC-Bedingungen: 30 m DB1, Temperaturprogramm mit 100°C Anfangstemperatur, 8°C/min Heizrate, 250 °C Endtemperatur).

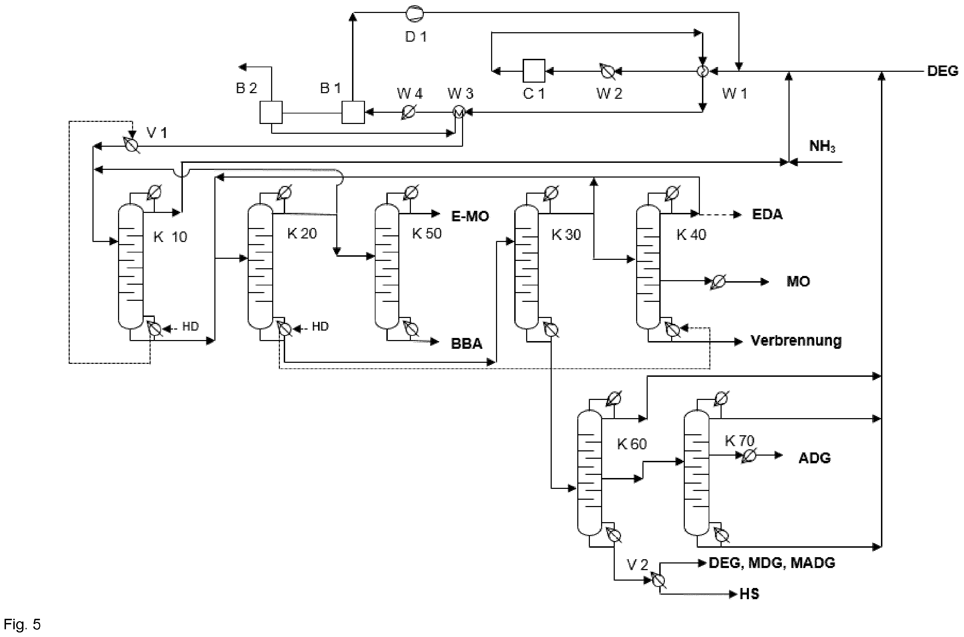

Eine besonders bevorzugte Ausführungsform des Verfahrens (siehe Abbildung 5) ist im Folgenden dargestellt.

Diglykol (DEG) wird mit dem Sumpfprodukt der Kolonne K70 (Hauptkomponenten Diglykol und Morpholyl-ADG) und den Kopfprodukten aus den Kolonnen K60 und K70 (Hauptkomponenten: Aminodiglykol, (2-Aminoethyl)morpholin und 2-(2-Aminoethoxy)ethylamin) gemischt und kontinuierlich dem Wärmetauscher W 1 zugeführt.

Flüssiges Ammoniak wird mit zurückgeführtem Ammoniak aus der Kolonne K10 gemischt und kontinuierlich dem Wärmetauscher W 1 zugeführt. Beide Ströme werden vor dem Wärmetauscher W 1 mit dem überwiegend aus Wasserstoff bestehendem Kreisgas vermischt. Das Kreisgas wird von dem am Syntheseausgang gelegenen Hochdruckabscheider B 1 mit dem Verdichter D 1 herangeführt. Vom Wärmetauscher W 1 wird das Gemisch durch einen Aufheizer W 2 erwärmt und zum Reaktor C 1 gefördert. Am dortigen Festbett-Katalysator erfolgt die Umsetzung des Diglykols zu Aminodiglykol und Morpholin. Der Reaktoraustrag wird dann in den Wärmetauschern W 1, W 3 und dem Luftkühler W 4 abgekühlt. In dem Hochdruckabscheider B 1 erfolgt die Trennung in Gas- und Flüssigphase. Die Gasphase wird -wie oben beschrieben - als Kreisgas zum Wärmetauscher W 1 geführt.

Die flüssige Phase wird aus dem Hochdruckabscheider B 1 in den Mitteldruckabscheider B 2 entspannt. Das dort frei werdende sogenannte Sprudelgas wird zur NH₃-Rückgewinnung in einen Absorber geleitet. Die zu ergänzende Wasserstoffmenge wird dem Netz entnommen und am Synthesezulauf eingespeist.

Aus dem Mitteldruckabscheider B 2 gelangt das Reaktionsgemisch dann über den Wärmetauscher W 3 in den Verdampfer V1.

### Ammoniakabtrennung (V1 und K10)

In V1 wird ein Teil des Ammoniaks aus dem Reaktionsgemisch abgetrennt. In der Kolonne K10 wird der restliche Ammoniak abdestilliert und auf den Reaktoreingang zurückgeführt. Darüber hinaus wird der Kolonne K10 ein Teil des Kopfproduktes der K20 zugeführt. Der im Verdampfer von K10 kondensierte Heizdampf wird mittels Entspannungsverdampfung wiederum verdampft und anschließend dem Verdampfer V1 zugeführt.

### Wasserabtrennung (K20)

In der Kolonne K20 wird das Reaktionswasser abgetrennt. Das Destillat, das überwiegend Wasser und wenige Leichtsieder (überwiegend Ethylmorpholin) enthält, wird zur Kolonne K50 geführt. Darüberhinaus wird der Kolonne K20 das Kopfprodukt der Morpholin-Reindestillation K40 (Hauptkomponenten: 1,2-Ethylendiamin, Morpholin und Wasser) und ein Teil des Kopfproduktes der K30 zugeführt. Der weitgehend wasserfreie Sumpf der K 20 (Hauptkomponenten: Morpholin, Aminodiglykol, Diglykol und hochsiedender Rückstand) wird zur Kolonne K30 entspannt. Der im Verdampfer von K20 kondensierte Heizdampf wird mittels Entspannungsverdampfung wiederum verdampft und anschließend dem Verdampfer der Kolonne K40 zugeführt.

### LS/HS-Abtrennung (K30)

In der Kolonne K30 wird der Sumpfabzug der Kolonne K20 in eine Leichtsiederfraktion (Hauptkomponente: Morpholin) und eine Hochsiederfraktion (Hauptkomponenten: Aminodiglykol, Diglykol und hochsiedender Rückstand) aufgetrennt. Der Sumpf wird der Kolonne K60 zugeführt. Das Kondensat wird der Kolonne K40 zugeführt.

### Morpholin-Reindestillation (K40)

In der Kolonne K40 wird Morpholin in einem gasförmigen Seitenabzug Morpholin und abgetrennt. Das Kopfdestillat (Hauptkomponenten: 1,2-Ethylendiamin, Morpholin und Wasser) wird zur K20 zurückgeführt bzw. nach Anreicherung von Ethylendiamin diskontinuierlich über einen Behälter ausgeschleust. Der Sumpf der Kolonne K40 (Morpholin und Methoxyethanol mit schwerer siedenden Nebenkomponenten) wird verbrannt. Der Verdampfer der K40 wird mit Heizdampf betrieben, der mittels Entspannungsverdampfung des im Verdampfer von K10 kondensierten Heizdampfs gewonnen wird.

### Ethylmorpholin-Destillation (K50)

In der Kolonne K50 wird N-Ethylmorpholin als Azeotrop mit Wasser aus dem Zulauf abgetrennt. Der Sumpf der Kolonne wird ausgeschleust.

### Rückstandsabtrennung (K60)

In der Kolonne K60 werden aus dem Zulauf Aminodiglykol und Diglykol gemeinsam als flüssiger Seitenabzug abgetrennt und zur Kolonne K70 gefördert. Das Destillat der Kolonne (Hauptkomponenten: Aminoethoxyethylamin, Aminoethylmorpholin, Aminodiglykol) wird auf den Reaktoreingang zurückgefördert. Der Sumpf der Kolonne wird dem Verdampfer (V2) zugeführt, wo Morpholinaminodiglykol (MADG), Morpholindiglykol (MDG) und Diethylenglykol (DEG) abgetrennt werden.

### Aminodiglykol-Destillation (K70)

In der Kolonne K70 wird aus dem Zulauf Aminodiglykol am Seitenabzug abgetrennt. Das Kondensat der Kolonne (Hauptkomponenten: Aminodiglykol, (2-Aminoethyl)morpholin und 2-(2-Aminoethoxy)ethylamin) wird auf den Reaktoreingang zurückgeführt. Der Sumpf der Kolonne wird ebenfalls auf den Reaktoreingang zurückgeführt.

Zu den Abbildungen:

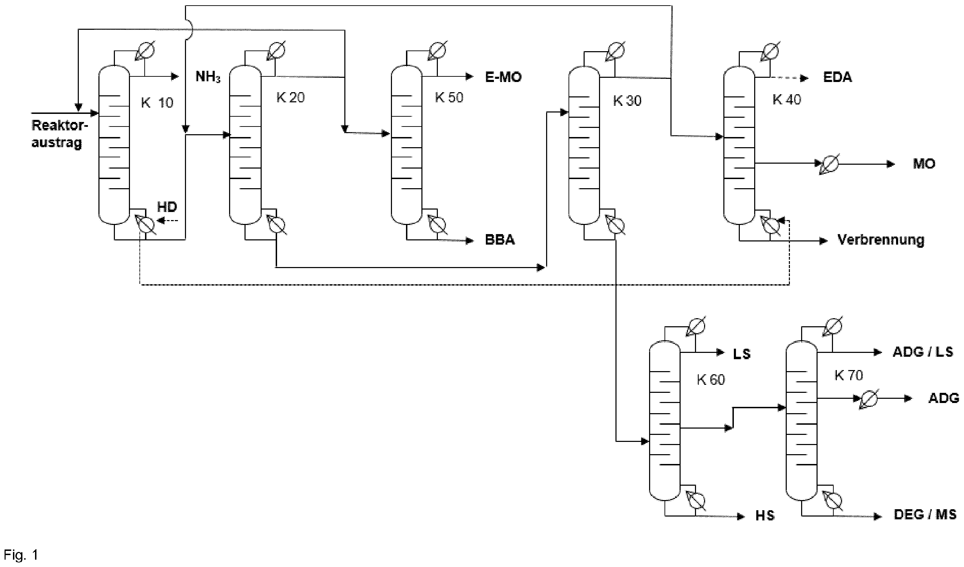

Abbildung 1 zeigt u.a. die erfindungsgemäße Gewinnung von MO und ADG durch eine 7-Kolonnen-Verschaltung. Zusätzlich ist exemplarisch die Wärmeintegration des Heizdampfs (HD) aus dem Verdampfer der K10 mittels Entspannungsverdampfung in den Verdampfer der K40 dargestellt. Zudem ist die teilweise Rückführung des Kopfproduktes der K20, in den Zulauf der Kolonne K10 sowie die teilweise Rückführung des Kopfproduktes der K30 in den Zulauf der der Kolonne K20 dargestellt.

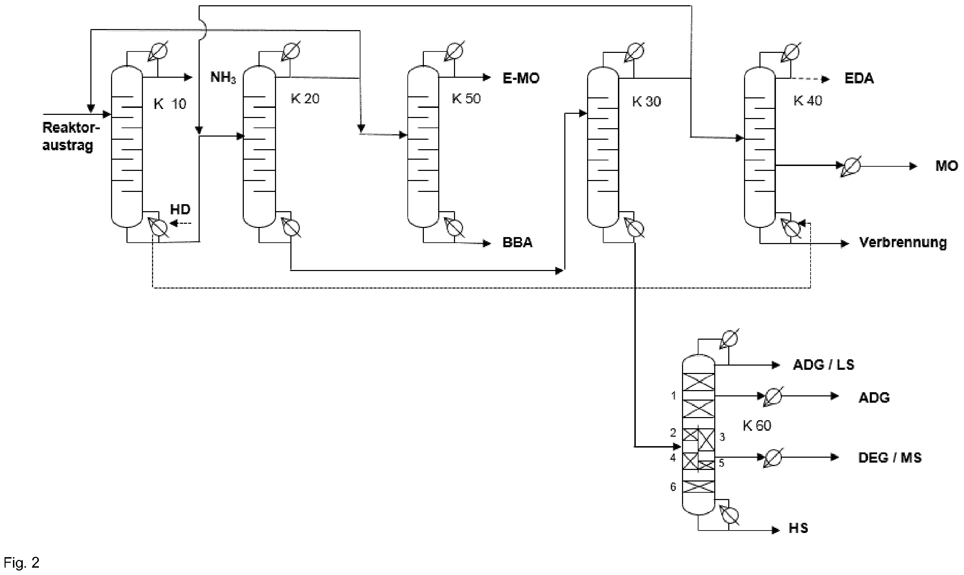

Abbildung 2 zeigt u.a. den Ersatz der Kolonnen K60 - K 70 der 7-Kolonnen-Verschaltung durch eine Trennwandkolonne (TK). Zusätzlich ist exemplarisch die Wärmeintegration des Heizdampfs (HD) aus dem Verdampfer der K10 mittels Entspannungsverdampfung in den Verdampfer der K40 dargestellt. Zudem ist die teilweise Rückführung des Kopfproduktes der K20, in den Zulauf der Kolonne K10 sowie die teilweise Rückführung des Kopfproduktes der K30 in den Zulauf der der Kolonne K20 dargestellt.

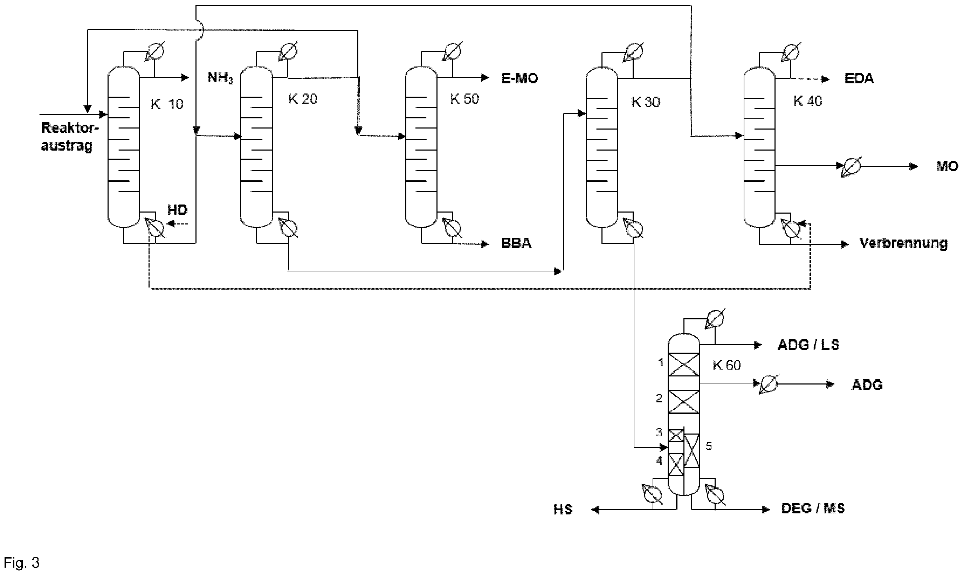

Abbildung 3 zeigt u.a. eine besondere Ausführungsform der Trennwandkolonne K60, in der die Trennwand (T) bis zum Boden der Kolonne ausgebildet ist. Zusätzlich ist exemplarisch die Wärmeintegration des Heizdampfs (HD) aus dem Verdampfer der K10 mittels Entspannungsverdampfung in den Verdampfer der K40 dargestellt. Zudem ist die teilweise Rückführung des Kopfproduktes der K20, in den Zulauf der Kolonne K10 sowie die teilweise Rückführung des Kopfproduktes der K30 in den Zulauf der der Kolonne K20 dargestellt.

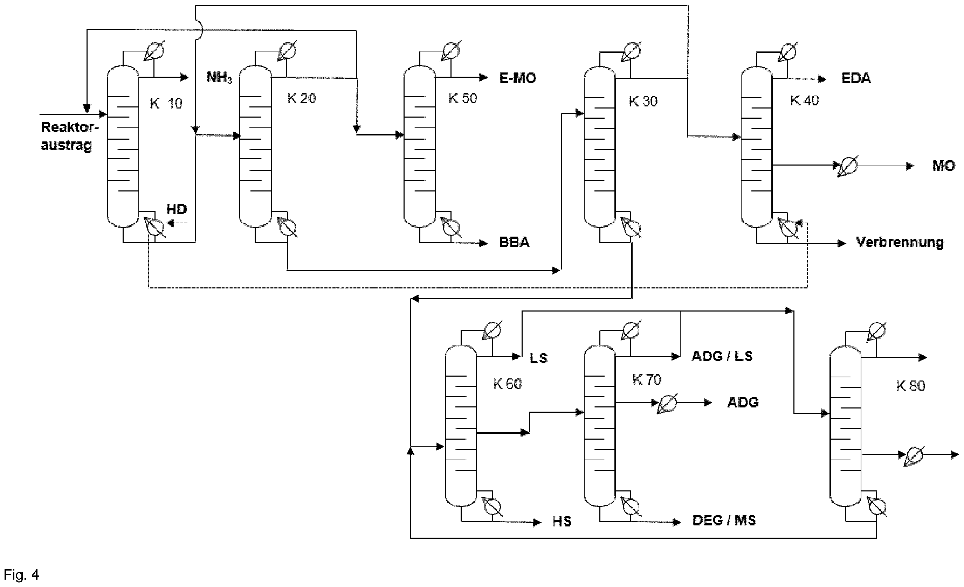

Abbildung 4 zeigt u.a. die erfindungsgemäße Gewinnung von MO und ADG durch eine 8-Kolonnen-Verschaltung. Zusätzlich ist exemplarisch die Wärmeintegration des Heizdampfs (HD) aus dem Verdampfer der K10 mittels Entspannungsverdampfung in den Verdampfer der K40 dargestellt. Zudem ist die teilweise Rückführung des Kopfproduktes der K20, in den Zulauf der Kolonne K10 sowie die teilweise Rückführung des Kopfproduktes der K30 in den Zulauf der der Kolonne K20 dargestellt.
Abbildung 5 zeigt die oben dargestellte besonders bevorzugte Ausführungsform des erfindungsgemäßen Verfahrens. HD = Heizdampf, HS = Hochsieder, LS = Leichtsieder, MS = Mittelsieder, BBA = behandlungsbedürftiges Abwasser.

Die Wärmeintegration mittels Entspannungsverdampfung ist in den Abbildungen 1 bis 5 mittels einer gestrichelten Linie dargestellt.

### Beispiele

Die nachfolgenden Beispiele beruhen auf Simulationsergebnissen, die mit der Software Aspen Plus der Firma Aspen Technology, Inc. erzielt wurden. Die in dem Programm verwendeten thermodynamischen Parameter für die einzelnen Reaktionsprodukte basieren auf veröffentlichten thermodynamischen Daten bzw. eigenen Messungen. Die Spezifizierung und die Simulation der angegebenen, verwendeten Destillationskolonnen erfolgte mit den üblichen, in der Software erhaltenen Routinen.

Zur Optimierung des Simulationsmodells wurden die simulierten Ergebnisse mit experimentellen Ergebnissen, soweit vorhanden, verglichen und das Simulationsmodel an die experimentellen Ergebnisse angepasst, so dass eine gute Übereinstimmung zwischen Simulation und experimentellen Daten erzielt werden konnte.

Die nachfolgenden Beispiele wurden mit dem optimierten Simulationsmodell berechnet.

Sofern nichts anderes angegeben, beziehen sich alle Druckangaben in den hier aufgeführten Beispielen auf den Absolutdruck.

Beispiel 1 (Reindestillation von Morpholin)

| | | **2,2 bar** | **500 mbar** | **200 mbar** |
|---|---|---|---|---|
| Zulauf | kg/h | 1973 | 1767 | 1767 |
| Rücklauf | kg/h | 2484 | 7795 | 5650 |
| Abzug (Destillat) | kg/h | 120 | 120 | 120 |
| Seitenabzug | kg/h | 1643 | 1630 | 1630 |
| Abzug (Sumpf) | kg/h | 210 | 17 | 17 |
| Sumpftemperatur | °C | 160,5 | 119 | 100 |
| Verdampfer | kW | 845 | 1588 | 1085 |
| Zusammensetzung Reinmorpholin | | | | |
| Morpholin | Gew.-% | 99,61 | 99,76 | 99,75 |
| 1,2-EDA | Gew.-% | 0,18 | 0,18 | 0,18 |
| Methoxyethanol | Gew.-% | 0,13 | 0,03 | 0,03 |
| E-MO | Gew.-% | 0,07 | 0,02 | 0,03 |
| Wasser | Gew.-% | - | - | |
| AEOEA | Gew.-% | 0,01 | 0,01 | 0,01 |
| Summe Gew.-% | | 100 | 100 | 100 |
| Zusammensetzung Zulauf K40 | | | | |
| Morpholin | Gew.-% | 93,93 | 95,3 | 98,04 |
| 1,2-EDA | Gew.-% | 5,6 | 4,14 | 1,33 |
| Methoxyethanol | Gew.-% | 0,12 | 0,12 | 0,12 |
| E-MO | Gew.-% | 0,08 | 0,07 | 0,07 |
| Wasser | Gew.-% | 0,06 | 0,07 | 0,07 |
| AEOEA | Gew.-% | 0,21 | 0,3 | 0,37 |
| Summe Gew.-% | | 100 | 100 | 100 |
| Zusammensetzung Sumpf K40 | | | | |
| Morpholin | Gew.-% | 97,8 | 53,8 | 47,2 |
| Methoxyethanol | Gew.-% | 0,15 | 9,5 | 9,6 |
| E-MO | Gew.-% | 0,2 | 4,8 | 3,9 |
| AEOEA | Gew.-% | 1,85 | 31,9 | 39,3 |
| Summe Gew.-% | | 100 | 100 | 100 |
| Zusammensetzung Destillat K40 | | | | |
| Morpholin | Gew.-% | 9 | 40,2 | 81,9 |
| 1,2-EDA | Gew.-% | 90 | 58,8 | 17,1 |
| Wasser | Gew.-% | 1 | 1 | 1 |
| Summe Gew.-% | | 100 | 100 | 100 |

Abkürzungen:

| | |
|---|---|
| 1,2-EDA: | 1,2-Ethylendiamin |
| E-MO: | N-Ethyl-morpholin |
| AEOEA: | Aminoethoxyethylamin |

Diskussion der Ergebnisse:
In obiger Tabelle sind die Ergebnisse der Reindestillation von Morpholin bei einem Druck von 2,2 bar, 500 mbar und 200 mbar dargestellt. Im erfindungsgemäßen Druckbereich (200 und 500 mbar) ist eine deutlich verbesserte Abtrennbarkeit von Methoxyethanol möglich. So beträgt der Gehalt an Methoxyethanol im Reinmorpholin lediglich noch 0,03 Gew.-% wohingegen er beim nicht-erfindungsgemäßen Druck (2,2 bar) deutlich höher, nämlich bis 0,13 Gew.-%, liegt.

Durch das Arbeiten im erfindungsgemäßen Druckbereich steigt der Energiebedarf im Verdampfer von 845 kW bei 2,2 bar auf 1588 kW bzw. 1085 kW bei 500 bzw. 200 mbar. Gleichzeitig ist es aufgrund der verringerten Sumpftemperatur möglich, den Verdampfer mit Heizdampf zu betreiben, welcher einen niedrigeren Druck aufweist. So weist der zur Beheizung des Sumpfs eingesetzte Heizdampf bei einem Kopfdruck von 2,2 bar üblicherweise einen Druck von 16 bar oder mehr auf. Bei den erfindungsgemäßen niedrigeren Kopfdrücken reicht ein Heizdampf aus, der einen Druck von 1 bis 10 bar aufweist. Ein solcher lässt sich energieeffizienter Herstellen, insbesondere dann, wenn eine Wärmeintegration (Entspannungsverdampfung) zum Einsatz kommt.

Beispiel 2 (K10 und K20 jeweils mit und ohne Rückführung):
In der ersten unten dargestellten Tabelle sind die Simulationsergebnisse für eine Rückführung des Stroms, der bei der Kolonne K20 über Kopf abgetrennt wird, in K10 dargestellt.

In der zweiten unten dargestellten Tabelle sind die Simulationsergebnisse für eine Rückführung des Stroms der bei Kolonne K30 über Kopf abgetrennt wird beziehungsweise eine Rückführung des am Seitenabzug von K40 erhaltenen Stroms (Morpholin) in die K20 dargestellt.

Aus den dargestellten Ergebnissen lässt sich entnehmen, dass sich durch eine entsprechende Rückführung die Temperatur im Sumpf der K10 bzw. K20 verringern lässt.

Für K10 ergibt sich eine Absenkung der Sumpftemperatur von 236,5 °C auf 227 °C.

Für K20 ergibt sich eine Absenkung der Sumpftemperatur von 254 °C auf 228 °C.

| **K10** | | **ohne Rückführung** | | **mit Rückführung** | |
|---|---|---|---|---|---|
| Kopfdruck | bar | 15,8 | | 15,8 | |
| Abzug (Destillat) | kg/h | 5337 | | 5337 | |
| Abzug (Sumpf) | kg/h | 9070 | | 9600 | |
| Sumpftemp. | °C | 236,5 | | 227 | |
| Zulauf | kg/h | 14407 | | 14937 | |
| | | Zusammensetzung Zulauf | Zusammensetzung Sumpf | Zusammensetzung Zulauf | Zusammensetzung Sumpf |
| Ammoniak | Gew.-% | 37,06 | 0,03 | 35,75 | 0,03 |
| Wasser | Gew.-% | 7,20 | 11,44 | 10,47 | 16,29 |
| M-MO | Gew.-% | 0,01 | 0,01 | 0,01 | 0,02 |
| Et-MO | Gew.-% | 0,03 | 0,04 | 0,03 | 0,05 |
| Morpholin | Gew.-% | 8,68 | 13,78 | 8,37 | 13,03 |
| 1,2-EDA | Gew.-% | 0,01 | 0,01 | 0,01 | 0,01 |
| Methoxyethanol | Gew.-% | 0,02 | 0,03 | 0,02 | 0,03 |
| AEOEA | Gew.-% | 1,76 | 2,80 | 1,70 | 2,65 |
| AE-MO | Gew.-% | 0,13 | 0,21 | 0,13 | 0,20 |
| ADG | Gew.-% | 15,91 | 25,27 | 15,34 | 23,88 |
| DEG | Gew.-% | 24,46 | 38,85 | 23,59 | 36,71 |
| MS | Gew.-% | 4,37 | 6,94 | 4,21 | 6,55 |
| HS | Gew.-% | 0,37 | 0,59 | 0,36 | 0,56 |
| SUMME | Gew.-% | 100 | 100 | 100 | 100 |

| **K20** | | **ohne Rückführung** | | **mit Rückführung** | |
|---|---|---|---|---|---|
| Kopfdruck | bar | 4,4 | | 4,4 | |
| Abzug (Destillat) | kg/h | 1045 | | 1575 | |
| Abzug (Sumpf) | kg/h | 8145 | | 10346 | |
| Sumpftemp. | °C | 254 | | 228 | |
| Zulauf | kg/h | 9190 | | 11921 | |
| | | Zusammensetzung Zulauf | Zusammensetzung Sumpf | Zusammensetzung Zulauf | Zusammensetzung Sumpf |
| Ammoniak | Gew.-% | 0,03 | 0,00 | 0,02 | 0,00 |
| Wasser | Gew.-% | 11,31 | 0,02 | 13,13 | 0,02 |
| M-MO | Gew.-% | 0,01 | 0,00 | 0,01 | 0,00 |
| Et-MO | Gew.-% | 0,04 | 0,02 | 0,07 | 0,04 |
| Morpholin | Gew.-% | 14,63 | 16,49 | 29,40 | 33,85 |
| 1,2-EDA | Gew.-% | 0,27 | 0,30 | 0,21 | 0,24 |
| Methoxyethanol | Gew.-% | 0,03 | 0,03 | 0,06 | 0,06 |
| AEOEA | Gew.-% | 2,77 | 3,12 | 2,40 | 2,76 |
| AE-MO | Gew.-% | 0,21 | 0,24 | 0,18 | 0,20 |
| ADG | Gew.-% | 24,94 | 28,14 | 19,23 | 22,16 |
| DEG | Gew.-% | 38,34 | 43,26 | 29,56 | 34,06 |
| MS | Gew.-% | 6,85 | 7,73 | 5,28 | 6,08 |
| HS | Gew.-% | 0,58 | 0,66 | 0,45 | 0,52 |
| SUMME | Gew.-% | 100 | 100 | 100 | 100 |

In den Tabellen 1 und 2 verwendete Abkürzungen:

| | |
|---|---|
| M-MO: | Methyl-morpholin |
| Et-MO: | Ethyl-morpholin |
| 1,2-EDA: | 1,2-Ethylendiamin |
| AEOEA: | Aminoethoxyethylamin |
| AE-MO: | Aminoethyl-morpholin |
| ADG: | Aminodiglycol |
| DEG: | Diethylenglycol |
| MS: | Mittelsieder |
| HS: | Hochsieder |

## Patentansprüche

1. Verfahren zur kontinuierlichen destillativen Auftrennung von Gemischen enthaltend Morpholin (MO), Monoaminodiglykol (ADG), Ammoniak, Wasser und Methoxyethanol (MOE), erhalten durch Umsetzung von Diethylenglykol (DEG) mit Ammoniak **dadurch gekennzeichnet, dass** Ammoniak, Wasser, ADG und DEG destillativ abgetrennt werden und der resultierende MO und MOE enthaltende Strom einer Destillationskolonne K40 zugeführt wird, in der bei einem Kopfdruck von 20 bis 2000 mbar über Sumpf MO, MOE und organische Produkte mit einem Siedepunkt ≥ 128 °C (1,013 bar) und über Kopf organische Produkte mit einem Siedepunkt ≤ 128 °C abgetrennt werden, sowie über einen Seitenabzug MO abgetrennt wird, wobei K40 mit einem Verdampfer zur Beheizung des Sumpfs ausgestattet ist, in den Heizdampf eingespeist wird, der einen Druck von 1 bis 10 bar aufweist.

2. Verfahren nach Anspruch 2, **dadurch gekennzeichnet, dass** zur destillativen Abtrennung von Ammoniak, Wasser, ADG und DEG der Ammoniak in einer ersten Destillationskolonne K10 über Kopf Ammoniak abgetrennt wird,
der Sumpfaustrag von K10 einer zweiten Destillationskolonne K20 zugeführt wird, in der Wasser und organische Produkte über Kopf bei einer Kopftemperatur von 45 bis 198 °C und einem Kopfdruck von 0,1 bis 15 bar abgetrennt werden,
der Sumpfaustrag von K20 einer dritten Destillationskolonne K30 zugeführt wird, in der MO, MOE und organische Produkte mit einem Siedepunkt < 140 °C (1,013 bar) (MO und MOE enthaltender Strom) über Kopf oder einen Seitenabzug abgetrennt werden und ADG, DEG und organische Produkte mit einem Siedepunkt > 190 °C (1,013 bar) über Sumpf abgetrennt werden.

3. Verfahren nach einem der vorhergehenden Ansprüche, **dadurch gekennzeichnet, dass** die Umsetzung von Diethyleneglykol (DEG) mit Ammoniak in Gegenwart von Wasserstoff und eines Heterogen-Hydrierkatalysators erfolgt, wobei
- der Umsatz bezogen auf DEG 40 bis 75 % beträgt,
- die Umsetzung bei einem Druck von 100 bis 300 bar und einer Temperatur von 170 °C bis 220 °C erfolgt,
- das Molverhältnis von Ammoniak zu DEG 4 bis 10 beträgt, und
- die Katalysatorbelastung im Bereich von 0,05 bis 5 kg Diethylenglykol (DEG) pro Liter Katalysator (Schüttvolumen) und Stunde liegt.

4. Verfahren nach dem vorhergehenden Anspruch, **dadurch gekennzeichnet, dass** der Wasser und organische Produkte enthaltende Strom, der bei der Kolonne K20 über Kopf abgetrennt wird, teilweise in den Zulauf oder Sumpf der Kolonne K10 zurückgeführt wird.

5. Verfahren nach einem der Ansprüche 2 bis 4, **dadurch gekennzeichnet, dass** der Wasser und organische Produkte enthaltene Strom, der bei Kolonne K20 über Kopf abgetrennt wird, einer Destillationskolonne K50 zugeführt wird, in der wässrige N-Ethyl-morpholin-Lösung (wässrige E-MO-Lösung) über Kopf oder einen flüssigen Seitenabzug und Wasser über Sumpf abgetrennt wird.

6. Verfahren nach einem der Ansprüche 2 bis 5, **dadurch gekennzeichnet, dass** der Sumpfaustrag von K30 einer Destillationskolonne K60 zugeführt wird, in der ADG im Seitenabzug abgetrennt wird, organische Produkte mit einem Siedepunkt ≤ 224,8 °C (1,013 bar) über Kopf abgetrennt und organische Produkte mit einem Siedepunkt > 255 °C (1,013 bar) über Sumpf abgetrennt werden.

7. Verfahren nach dem vorhergehenden Anspruch, **dadurch gekennzeichnet, dass** der ADG enthaltene Strom, der bei K60 im Seitenabzug abgetrennt wird, einer Destillationskolonne K70 zugeführt wird, in der ADG über einen Seitenabzug abgetrennt wird, organische Produkte mit einem Siedepunkt ≥ 224,8 °C (1,013 bar) über Sumpf und organische Produkte mit einem Siedepunkt ≤ 224,8 (1,013 bar) über Kopf abgetrennt werden.

8. Verfahren nach Anspruch 6, **dadurch gekennzeichnet, dass** es sich bei der Kolonne K60 um eine Trennwandkolonne (TK) handelt.

9. Verfahren nach einem der Ansprüche 6 bis 8, **dadurch gekennzeichnet, dass** der oder die ADG enthaltende/n Strom/Ströme, der/die bei der Kolonne K60 und/oder K70 über Kopf abgetrennt wird/werden, ganz oder teilweise einer Kolonne K80 zugeführt wird/werden, in der ADG und organische Produkte mit einem Siedepunkt ≥ 224,8 °C (1,013 bar) über Sumpf und organische Produkte mit einem Siedepunkt ≤ 224,8 (1,013 bar) über Kopf abgetrennt werden.

10. Verfahren nach einem der vier vorhergehenden Ansprüche, **dadurch gekennzeichnet, dass** der organische Produkte enthaltende Strom, der bei Kolonne K60 über Sumpf abgetrennt wird, einem Verdampfer V2 zugeführt wird, in dem Morpholinaminodiglykol, Morpholindiglykol und DEG gasförmig abgetrennt werden.

11. Verfahren nach einem der vorhergehenden Ansprüche, **dadurch gekennzeichnet, dass** die Umsetzung von DEG mit Ammoniak in einem Reaktor C1 erfolgt, wobei DEG und Ammoniak vor Eintritt in C1 mittels eines Wärmetauschers W2 erhitzt werden, in den Heizdampf eingespeist wird, der einen Druck von 2 bis 50 bar aufweist.

12. Verfahren nach den Ansprüchen 2, 5, 6 und 7 sowie optional mindestens einem der Ansprüche 3, 4, und 8 bis 11, **dadurch gekennzeichnet, dass** die Kolonnen K10, K20, K30, K50, K60 und K70 jeweils mit einem Verdampfer zur Beheizung des Sumpfs ausgestattet sind, in den Heizdampf eingespeist wird, der einen Druck von 2 bis 50 bar aufweist.

13. Verfahren nach einem der vorhergehenden Ansprüche, **dadurch gekennzeichnet, dass** Heizdampf für die Kolonne K40 mittels Entspannungsverdampfung eines Kondensats erhalten wird, das aus der Kondensation eines Heizdampfs in einem Wärmetauscher resultiert, wobei der Heizdampf vor seiner Kondensation in dem Wärmetauscher einen Druck von 2 bis 50 bar aufweist.

14. Verfahren nach den drei vorhergehenden Ansprüchen, **dadurch gekennzeichnet, dass** es sich bei dem Wärmetauscher um den Wärmetauscher W2 oder den Verdampfer einer der Kolonnen K10, K20, K30, K50, K60 oder K70 handelt.

15. Verfahren nach einem der Ansprüche 2 bis 12, **dadurch gekennzeichnet, dass** das Reaktionsgemisch der Umsetzung von DEG mit Ammoniak vor Zuführung in die Kolonne K10 einem Verdampfer V1 zugeführt wird, in dem ein Teil des Ammoniaks gasförmig abgetrennt wird.

16. Verfahren nach dem vorhergehenden Anspruch, **dadurch gekennzeichnet, dass** in den Verdampfer V1 Heizdampf eingespeist wird, der einen Druck von 1 bis 10 bar aufweist.

17. Verfahren nach dem vorhergehenden Anspruch, **dadurch gekennzeichnet, dass** Heizdampf für den Verdampfer V1 mittels Entspannungsverdampfung eines Kondensats erhalten wird, das aus der Kondensation eines Heizdampfs in einem Wärmetauscher resultiert, wobei der Heizdampf vor seiner Kondensation in dem Wärmetauscher einen Druck von 2 bis 50 bar aufweist.

18. Verfahren nach Anspruch 11 und 12 sowie dem vorhergehenden Anspruch, **dadurch gekennzeichnet, dass** es sich beim dem Wärmetauscher, um den Wärmetauscher W2 oder den Verdampfer einer der Kolonnen K10, K20, K30, K50, K60 oder K70 handelt.

## Claims

1. A process for the continuous distillative separation of mixtures comprising morpholine (MO), monoaminodiglycol (ADG), ammonia, water and methoxyethanol (MOE), obtained by reacting diethylene glycol (DEG) with ammonia, wherein ammonia, water, ADG and DEG are removed by distillation and the resulting stream comprising MO and MOE is supplied to a distillation column K40 in which at a top pressure of from 20 to 2000 mbar MO, MOE and organic products having a boiling point ≥ 128°C (1.013 bar) are removed via the bottom and organic products having a boiling point ≤ 128°C are removed overhead, and also MO is removed via a side draw, where K40 is equipped with an evaporator for heating the bottoms, into which is fed heating vapor having a pressure of from 1 to 10 bar.

2. The process according to claim 2, wherein, for the distillative removal of ammonia, water, ADG and DEG, the ammonia is removed overhead ammonia in a first distillation column K10,
the bottoms output from K10 is supplied to a second distillation column K20, in which water and organic products are removed overhead at a top temperature of from 45 to 198°C and a top pressure of from 0.1 to 15 bar,
the bottoms output from K20 is supplied to a third distillation column K30, in which MO, MOE and organic products having a boiling point < 140°C (1.013 bar) (stream comprising MO and MOE) are removed overhead or via a side draw and ADG, DEG and organic products having a boiling point of > 190°C (1.013 bar) are removed via the bottom.

3. The process according to any of the preceding claims, wherein the reaction of diethylene glycol (DEG) with ammonia is effected in the presence of hydrogen and a heterogeneous hydrogenation catalyst, wherein
- the conversion based on DEG is 40% to 75%,
- the reaction is effected at a pressure of from 100 to 300 bar and a temperature of from 170°C to 220°C,
- the molar ratio of ammonia to DEG is 4 to 10, and
- the catalyst hourly space velocity is in the range from 0.05 to 5 kg of diethylene glycol (DEG) per liter of catalyst (bed volume) and per hour.

4. The process according to the preceding claim, wherein the stream comprising water and organic products which is removed overhead at column K20 is partially recycled into the feed or bottom of column K10.

5. The process according to any of claims 2 to 4, wherein the stream comprising water and organic products which is removed overhead at column K20 is supplied to a distillation column K50 in which aqueous N-ethylmorpholine solution (aqueous EMO solution) is removed overhead or via a liquid side draw and water is removed via the bottom.

6. The process according to any of claims 2 to 5, wherein the bottoms output from K30 is supplied to a distillation column K60 in which ADG is removed in the side draw, organic products having a boiling point ≤ 224.8°C (1.013 bar) are removed overhead and organic products having a boiling point > 255°C (1.013 bar) are removed via the bottom.

7. The process according to the preceding claim, wherein the ADG-comprising stream which is removed in the side draw at K60 is supplied to a distillation column K70 in which ADG is removed via a side draw, organic products having a boiling point ≥ 224.8°C (1.013 bar) are removed via the bottom and organic products having a boiling point ≤ 224.8 (1.013 bar) are removed overhead.

8. The process according to claim 6, wherein column K60 is a dividing wall column (DWC).

9. The process according to any of claims 6 to 8, wherein the ADG-comprising stream(s) which is/are removed overhead at column K60 and/or K70 is/are supplied wholly or partially to a column K80 in which ADG and organic products having a boiling point ≥ 224.8°C (1.013 bar) are removed via the bottom and organic products having a boiling point ≤ 224.8 (1.013 bar) are removed overhead.

10. The process according to any of the four preceding claims, wherein the stream comprising organic products which is removed via the bottom at column K60 is supplied to an evaporator V2 in which morpholine aminodiglycol, morpholine diglycol and DEG are removed in gaseous form.

11. The process according to any of the preceding claims, wherein the reaction of DEG with ammonia is effected in a reactor C1, wherein DEG and ammonia are heated prior to entry into C1 by means of a heat exchanger W2 into which is fed heating vapor having a pressure of from 2 to 50 bar.

12. The process according to claims 2, 5, 6 and 7 and also optionally at least one of claims 3, 4 and 8 to 11, wherein columns K10, K20, K30, K50, K60 and K70 are each equipped with an evaporator for heating the bottoms, into which is fed heating vapor having a pressure of from 2 to 50 bar.

13. The process according to any of the preceding claims, wherein heating vapor for the column K40 is obtained by means of flash evaporation of a condensate resulting from the condensation of a heating vapor in a heat exchanger, wherein the heating vapor prior to its condensation in the heat exchanger has a pressure of from 2 to 50 bar.

14. The process according to the three preceding claims, wherein the heat exchanger is the heat exchanger W2 or the evaporator of one of the columns K10, K20, K30, K50, K60 or K70.

15. The process according to any of claims 2 to 12, wherein the reaction mixture from the reaction of DEG with ammonia, prior to being supplied to column K10, is supplied to an evaporator V1 in which a portion of the ammonia is removed in gaseous form.

16. The process according to the preceding claim, wherein heating vapor having a pressure of from 1 to 10 bar is fed into the evaporator V1.

17. The process according to the preceding claim, wherein heating vapor for the evaporator V1 is obtained by means of flash evaporation of a condensate resulting from the condensation of a heating vapor in a heat exchanger, wherein the heating vapor prior to its condensation in the heat exchanger has a pressure of from 2 to 50 bar.

18. The process according to claims 11 and 12 and also the preceding claim, wherein the heat exchanger is the heat exchanger W2 or the evaporator of one of the columns K10, K20, K30, K50, K60 or K70.

## Revendications

1. Procédé pour la séparation par distillation en continu de mélanges contenant de la morpholine (MO), du monoaminodiglycol (ADG), de l'ammoniac, de l'eau et du méthoxyéthanol (MOE), obtenu par transformation de diéthylèneglycol (DEG) avec de l'ammoniac, **caractérisé en ce que**, de l'ammoniac, de l'eau, de l'ADG et du DEG sont séparés par distillation et le flux résultant contenant du MO et du MOE est alimenté à une colonne de distillation K40, dans laquelle, à une pression de tête de 20 à 2 000 mbars, du MO, du MOE et des produits organiques dotés d'un point d'ébullition ≥ 128 °C (1,013 bar) sont séparés par le fond et des produits organiques dotés d'un point d'ébullition ≤ 128 °C sont séparés par la tête, du MO étant également séparé par un soutirage latéral, K40 étant équipé d'un évaporateur pour le chauffage du fond, dans lequel de la vapeur de chauffe est introduite, qui présente une pression de 1 à 10 bars.

2. Procédé selon la revendication 2, **caractérisé en ce que** pour la séparation par distillation d'ammoniac, d'eau, d'ADG et de DEG, l'ammoniac est séparé dans une première colonne de distillation K10 par la tête ammoniac,
l'évacuation de fond de K10 est alimentée à une deuxième colonne de distillation K20, dans laquelle de l'eau et des produits organiques sont séparés par la tête à une température de tête de 45 à 198 °C et à une pression de tête de 0,1 à 15 bars,
l'évacuation de fond de K20 est alimentée à une troisième colonne de distillation K30, dans laquelle du MO, du MOE et des produits organiques dotés d'un point d'ébullition < 140 °C (1,013 bar) (flux contenant du MO et du MOE) sont séparés par la tête ou par un soutirage latéral et de l'ADG, du DEG et des produits organiques dotés d'un point d'ébullition > 190 °C (1,013 bar) sont séparés par le fond.

3. Procédé selon l'une quelconque des revendications précédentes, **caractérisé en ce que** la transformation de diéthylèneglycol (DEG) avec de l'ammoniac est réalisée en présence d'hydrogène et d'un catalyseur d'hydrogénation hétérogène,
- la conversion étant de 40 à 75 % par rapport au DEG,
- la transformation étant réalisée à une pression de 100 à 300 bars et à une température de 170 °C à 220 °C,
- le rapport molaire d'ammoniac sur DEG étant de 4 à 10, et
- la charge de catalyseur se situant dans la plage de 0, 05 à 5 kg de diéthylèneglycol (DEG) par litre de catalyseur (volume apparent) et par heure.

4. Procédé selon la revendication précédente, **caractérisé en ce que** le flux contenant de l'eau et des produits organiques qui est séparé par la tête dans la colonne K20, est recyclé partiellement dans l'arrivée ou le fond de la colonne K10.

5. Procédé selon l'une quelconque des revendications 2 à 4, **caractérisé en ce que** le flux contenant de l'eau et des produits organiques qui est séparé par la tête dans la colonne K20, est alimenté à une colonne de distillation K50, dans laquelle une solution aqueuse de N-éthyl-morpholine (solution aqueuse d'E-MO) est séparée par la tête ou un soutirage latéral liquide et de l'eau est séparée par le fond.

6. Procédé selon l'une quelconque des revendications 2 à 5, **caractérisé en ce que** l'évacuation de fond de K30 est alimentée à une colonne de distillation K60, dans laquelle de l'ADG est séparé dans le soutirage latéral, des produits organiques dotés d'un point d'ébullition ≤ 224,8 °C (1,013 bar) sont séparés par la tête et des produits organiques dotés d'un point d'ébullition > 255 °C (1,013 bar) sont séparés par le fond.

7. Procédé selon la revendication précédente, **caractérisé en ce que** le flux contenant de l'ADG, qui est séparé dans K60 dans le soutirage latéral, est alimenté à une colonne de distillation K70, dans laquelle de l'ADG est séparé par un soutirage latéral, des produits organiques dotés d'un point d'ébullition ≥ 224,8 °C (1,013 bar) sont séparés par le fond et des produits organiques dotés d'un point d'ébullition ≤ 224,8 (1,013 bar) sont séparés par la tête.

8. Procédé selon la revendication 6, **caractérisé en ce que** la colonne K60 est une colonne à paroi de séparation (TK).

9. Procédé selon l'une quelconque des revendications 6 à 8, **caractérisé en ce que** le ou les flux contenant de l'ADG qui sont séparés par la tête dans la colonne K60 et/ou K70, sont alimentés entièrement ou partiellement à une colonne K80, dans laquelle de l'ADG et des produits organiques dotés d'un point d'ébullition ≥ 224,8 °C (1,013 bar) sont séparés par le fond et des produits organiques dotés d'un point d'ébullition ≤ 224,8 (1,013 bar) sont séparés par la tête.

10. Procédé selon l'une quelconque des quatre revendications précédentes, **caractérisé en ce que** le flux contenant des produits organiques qui est séparé dans la colonne K60 par le fond, est alimenté à un évaporateur V2 dans lequel du morpholinaminodiglycol, du morpholinediglycol et du DEG sont séparés en phase gazeuse.

11. Procédé selon l'une quelconque des revendications précédentes, **caractérisé en ce que** la transformation de DEG avec de l'ammoniac est réalisée dans un réacteur C1, le DEG et l'ammoniac étant chauffés avant l'entrée dans C1 au moyen d'un échangeur de chaleur W2, dans lequel de la vapeur de chauffe est introduite, qui présente une pression de 2 à 50 bars.

12. Procédé selon les revendications 2, 5, 6 et 7 ainsi qu'éventuellement au moins l'une quelconque des revendications 3, 4, et 8 à 11, **caractérisé en ce que** les colonnes K10, K20, K30, K50, K60 et K70 sont à chaque fois équipées d'un évaporateur pour le chauffage du fond, dans lequel de la vapeur de chauffe est introduite qui présente une pression de 2 à 50 bars.

13. Procédé selon l'une quelconque des revendications précédentes, **caractérisé en ce que** la vapeur de chauffe pour la colonne K40 est obtenue au moyen de l'évaporation de détente d'un condensat, qui résulte de la condensation d'une vapeur de chauffe dans un échangeur de chaleur, la vapeur de chauffe, avant sa condensation dans l'échangeur de chaleur, présentant une pression de 2 à 50 bars.

14. Procédé selon les trois revendications précédentes, **caractérisé en ce que** l'échangeur de chaleur est l'échangeur de chaleur W2 ou l'évaporateur de l'une des colonnes K10, K20, K30, K50, K60 ou K70.

15. Procédé selon l'une quelconque des revendications 2 à 12, **caractérisé en ce que** le mélange réactionnel de la transformation de DEG avec de l'ammoniac est alimenté, avant l'alimentation dans la colonne K10, à un évaporateur V1 dans lequel une partie de l'ammoniac est séparée sous forme gazeuse.

16. Procédé selon la revendication précédente, **caractérisé en ce que** de la vapeur de chauffe est introduite dans l'évaporateur V1, qui présente une pression de 1 à 10 bars.

17. Procédé selon la revendication précédente, **caractérisé en ce que** la vapeur de chauffe pour l'évaporateur V1 est obtenue au moyen de l'évaporation de détente d'un condensat, qui résulte de la condensation d'une vapeur de chauffe dans un échangeur de chaleur, la vapeur de chauffe, avant sa condensation dans l'échangeur de chaleur, présentant une pression de 2 à 50 bars.

18. Procédé selon les revendications 11 et 12 ainsi que la revendication précédente, **caractérisé en ce que** l'échangeur de chaleur est l'échangeur de chaleur W2 ou l'évaporateur de l'une des colonnes K10, K20, K30, K50, K60 ou K70.
